# FASCICULE DE BREVET EUROPEEN

(11) **EP 0 892 633 B1**
(45) Date de publication et mention de la délivrance du brevet: **05.03.2003**
(21) Numéro de dépôt: 97911304.0
(22) Date de dépôt: 20.10.1997
(51) Int. Cl.: A61K 7/48, A61K 31/07

(54) **UTILISATION DES INHIBITEURS DE L'ACTIVITE DE L'ACIDE RETINOIQUE POUR TRAITER LES PEAUX SENSIBLES ET/OU LES DOMMAGES AIGUS INDUITS PAR LES RAYONNEMENTS U.V.**
VERWENDUNG VON INHIBITOREN DER RETINOSÄUREAKTIVITÄT ZUR BEHANDLUNG EMPFINDLICHER HAUT UND/ODER DURCH UV STRAHLUNG BEWIRKTER AKUTER SCHÄDIGUNGEN
USE OF INHIBITORS OF THE ACTIVITY OF RETINOIC ACID FOR TREATING SENSITIVE SKIN AND/OR ACUTE DAMAGE INDUCED BY UV RADIATION

(30) Priorité: 31.10.1996 FR 9613362
(43) Date de publication de la demande: 27.01.1999
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: DEMARCHEZ, Michel, F-06620 Le Bar sur Loup (FR); JOMARD, André, F-06460 Saint Vallier de Thiey (FR)
(74) Mandataire: Tezier Herman, Béatrice
(86) Numéro de dépôt international: FR9701879
(87) Numéro de publication internationale: WO98018440

(56) Documents cités:
- EP-A- 0 740 937
- WO-A-96/30009

## Description

La présente invention concerne une utilisation d'au moins un inhibiteur de l'activité de l'acide rétinoïque dans une composition cosmétique ou pour la fabrication d'une composition pharmaceutique, l'inhibiteur de l'activité de l'acide rétinoïque ou la composition pharmaceutique étant destiné à traiter les peaux sensibles et/ou les dommages aigus induits par les rayonnements U.V..

Il est connu que certaines peaux sont plus sensibles que d'autres.

Les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettent pas de caractériser les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques ou dermatologiques l'emploi de produits à caractère irritant tels que des tensioactifs, des conservateurs, des solvants, des propulseurs ou des parfums.

Il a été depuis réalisé de nombreux tests cliniques et il a ainsi été déterminé les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, échauffements, inconforts, tiraillements, etc.

On a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

Les peaux sensibles peuvent être scindées en deux grandes formes cliniques, les peaux irritables et/ou réactives et les peaux intolérantes.

Une peau irritable et/ou réactive est une peau qui réagit par un prurit, c'est-à-dire par des démangeaisons, ou par des picotements, à différents facteurs tels que l'environnement (exposition aux rayons UV trop importante), les émotions, les aliments, le vent, les frottements, le rasoir, le savon, les tensioactifs, l'eau dure à forte concentration de calcaire, les variations de température ou la laine.

Une peau intolérante est une peau qui réagit par des sensations d'échauffement, de tiraillements, de fourmillements et/ou de rougeurs, à différents facteurs tels que l'environnement (exposition aux rayons UV trop importante), les émotions, les aliments.

Une exposition trop importante de la peau aux rayons UV peut conduire à des dommages cutanés aigus, tels qu'une peau érythémateuse et/ou irritée et/ou rouge et/ou gonflée et/ou oedémateuse et/ou desquamante. Ces dommages cutanés aigus aboutissent à ce que l'on appelle communément "coup de soleil". Ces dommages aigus sont à distinguer des dommages chroniques, tels que les pathologies cancéreuses ou une peau photovieillie, qui correspond principalement à des dommages dermiques, et subsidiairement à des dommages épidermiques.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Les produits à caractère irritant peuvent être utilisés dans des compositions cosmétiques ou pharmaceutiques, et plus particulièrement dermatologiques, bien entendu pour d'autres effets. Ainsi, ils sont généralement utilisés en tant qu'agents actifs, tensioactifs, conservateurs, parfums, solvants ou propulseurs desdites compositions.

Mais du fait de cet effet indésirable, ces produits sont généralement utilisés en des doses très faibles. L'utilisation à faible quantité de ces produits peut alors s'avérer peu avantageuse par rapport à l'utilisation d'autres produits moins actifs, mais moins ou pas irritants et donc utilisés en plus grande quantité.

Par conséquent, il existe un besoin dans le domaine cosmétique et pharmaceutique de trouver un moyen permettant d'utiliser ces produits, sans que ces derniers présentent un caractère irritant reprochable par l'utilisateur.

On sait, d'une manière générale, que l'acide rétinoïque tout-trans (all-trans retinoic acid) agit sur la différenciation et/ou la prolifération des cellules en interagissant avec des récepteurs nucléaires ou RARs (Retinoic Acid Receptors) contenus dans le noyau cellulaire. De nombreux analogues structuraux synthétiques de l'acide rétinoïque tout-trans ou de l'acide 9-cis-rétinoïque, couramment dénommés "rétinoïdes", ont été décrits à ce jour dans la littérature. Il existe, à ce jour, trois sous-types identifiés de récepteurs RAR appelés respectivement RAR-α, RAR-β et RAR-γ. Ces récepteurs, après fixation du ligand (i.e. de l'acide rétinoïque tout-trans), interagissent avec la région promotrice de gènes régulés par l'acide rétinoïque au niveau d'éléments de réponses spécifiques (RARE).

Certains analogues peuvent se fixer et activer un sous-type de récepteur RAR (α, β ou γ) particulier D'autres analogues, enfin, ne présentent aucune activité sélective particulière vis-à-vis de ces différents récepteurs. A cet égard, et par exemple, l'acide rétinoïque tout-trans active les RARs (ligand agoniste spécifique RARs), tous sous-types confondus.

L'acide rétinoïque et les rétinoïdes en général ont été revendiqués pour traiter les désordres ou affections suivants: les acnés vulgaires, comédoniennes, polymorphes, rosacées, les acnés nodulokystiques, conglobata, les acnés séniles, les acnés secondaires telles que l'acné solaire, médicamenteuse ou professionnelle; les autres types de troubles de la kératinisation, notamment les ichtyoses, les états, ichtyosiformes, la maladie de Darrier, les kératodermies palmoplantaires, les leucoplasies et les états leucoplasiformes, le lichen cutané ou muqueux (buccal); les autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les dommages induits par les rayonnements U.V. ; le vieillissement de la peau, qu'il soit photo-induit ou chronologique ou les pigmentations et les kératoses actiniques, ou toutes pathologies associées au vieillissement chronologique ou actinique ; les troubles de la cicatrisation ou les vergetures ; les troubles de la fonction sébacée tels que l'hyperséborrhée de l'acné ou la séborrhée simple.

Il a également été décrit dans la demande de brevet WO 96/30009 l'association de composés agonistes des RAR-α et RAR-γ avec des antagonistes RAR-α pour traiter des peaux photovieillies, en spécifiant que les antagonistes RAR-α seuls ne sont pas actifs pour ce type de traitement.

L'acide rétinoïque et les rétinoïdes en général, en se liant avec les récepteurs RARs, permettent de réguler l'activité des récepteurs RARs et de traiter les désordres ou affections ci-dessus.

En ce qui concerne les atopies cutanées, il est connu qu'elles présentent une composante immunologique.

Ainsi, on pouvait donc penser que les inhibiteurs de l'activité de l'acide rétinoïque ne présentaient aucune activité sur la peau sensible, telle que définie ci-dessus, et sur les dommages induits par les rayonnements U.V..

On sait que l'acide rétinoïque et ses analogues (appelés également rétinoïdes) sont capables d'induire la différenciation des cellules (F9) de tératocarcinome embryonnaire de souris. La sécrétion de l'activateur plasminogène qui accompagne cette différenciation est un indice de la réponse biologique des cellules F9 aux rétinoïdes. On sait également que la capacité de ces rétinoïdes à induire l'activateur plasminogène est corrélée directement avec l'affinité qu'ils ont pour les récepteurs RARs endogènes aux cellules F9 (Skin pharmacol. 1990, 3, pp.256-267).

Les antagonistes de l'acide rétinoïque inhibent l'activité de l'acide rétinoïque ou ses métabolites au niveau cellulaire. Il s'agit plus particulièrement des antagonistes RARs qui viennent se lier aux récepteurs RARs, mais n'induisent pas l'activité observée pour l'acide rétinoïque ou les rétinoïdes, telle que celle observée sur les cellules F9.

Ainsi, il a été montré que des antagonistes des récepteurs RARα inhibent la différenciation cellulaire induite par les rétinoïdes sur les cellules de la lignée cellulaire HL60 ou au contraire réversent l'inhibition de la prolifération des cellules-B de la souris induite par les rétinoïdes (C. APFEL & al., Proc. Natl. Acad. Sci. USA, 89, 1992, 7129-7133).

La demande de brevet EP-A-0 740 937 divulgue l'utilisation de composés de type rétinoïdes en tant qu'agents actifs dans une composition cosmétique ou pour la fabrication d'une composition pharmaceutique, ces compositions étant destinées au traitement de désordres et/ou d'affections liés à une surrégulation des récepteurs RARs et/ou à une hypervitaminose A.

Un des buts de la présente invention est donc de proposer des composés qui peuvent traiter les peaux sensibles.

Un autre but est de proposer des composés qui peuvent notamment permettre d'utiliser des produits dans des compositions cosmétiques ou pharmaceutiques, plus particulièrement dermatologiques, sans que ces produits présentent un caractère irritant reprochable par l'utilisateur.

Un autre but est de proposer un traitement des dommages cutanés aigus dus à une exposition aux rayonnements U.V..

Ces buts et d'autres sont atteints par la présente invention qui concerne l'utilisation d'au moins un inhibiteur de l'activité de l'acide rétinoïque dans une composition cosmétique ou pour la préparation d'une composition pharmaceutique, l'inhibiteur de l'activité de l'acide rétinoïque ou la composition pharmaceutique étant destiné à traiter les peaux sensibles et/ou les dommages cutanés aigus induits par les rayonnements U.V..

La composition pharmaceutique est de préférence une composition dermatologique.

Les peaux sensibles et/ou les dommages cutanés aigus induits par les rayonnements U.V. sont tels que définis précédemment.

Dans le cadre de la présente invention, le traitement peut être réalisé de manière préventive ou curative. Dans les cas des dommages cutanés aigus induits par les rayonnements U.V., il est réalisé, de préférence, de manière curative.

La présente invention concerne également l'utilisation d'au moins un inhibiteur de l'activité de l'acide rétinoïque dans une composition cosmétique ou pour la préparation d'une composition pharmaceutique, l'inhibiteur de l'activité de l'acide rétinoïque ou la composition pharmaceutique étant destiné à traiter les irritations cutanées et/ou les érythèmes et/ou les rougeurs et/ou les sensations dysesthésiques et/ou les sensations d'échauffement et/ou les prurits et/ou les picotements et/ou les fourmillements et/ou les démangeaisons et/ou les tiraillements de la peau.

Plus particulièrement, l'inhibiteur de l'activité de l'acide rétinoïque ou la composition pharmaceutique est destiné à traiter les érythèmes et/ou les irritations et/ou les rougeurs et/ou les gonflements et/ou les oedèmes et/ou la desquamation dus aux rayonnements U.V..

Les inhibiteurs de l'activité de l'acide rétinoïque peuvent agir selon deux voies, la première en accélérant le métabolisme cellulaire de l'acide rétinoïque de manière à diminuer la concentration cellulaire de ce dernier, la seconde en antagonisant son action au niveau cellulaire.

Les inhibiteurs de l'activité de l'acide rétinoïque peuvent donc être selon l'invention des accélérateurs du métabolisme de l'acide rétinoïque ou des antagonistes, agonistes inverses ou agonistes partiels de l'acide rétinoïque.

Par antagonistes de l'acide rétinoïque, on entend de préférence selon l'invention les composés qui inhibent l'action de l'acide rétinoïque et/ou de ses métabolites et/ou des retinoïdes. Il s'agit plus particulièrement des antagonistes RARs, qui se fixent aux récepteurs RARs sans toutefois les activer. Ainsi, ils n'induisent pas la différenciation de ces cellules F9, mais se lient cependant aux RARs, cette liaison étant du type antagoniste. Le test F9 est celui décrit dans l'article de Skin pharmacol. 1990, 3, pp.256-267.

La faculté d'un composé de se lier aux RARs est déterminée au moyen de tests classiques pour l'homme de l'art. Ces tests sont notamment décrits dans les références suivantes : (1) "Sélective Synthetic Ligands for Nuclear Retinoic Acid Receptor Subtypes" *in* RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" *in* Pharmacol Skin, Basel, Karger, 1993, Volume. 5, pp 117-127 ; (3) "Selective Synthetic Ligands for Human Nuclear Retinoic Acid Receptors" *in* Skin Pharmacology, 1992, Vol. 5, pp 57-65 ; (4) "Identification of Synthetic Retinoids with Selectivity for Human Nuclear Retinoic Acid Receptor-γ" *in* Biochemical and Biophysical Research Communications, Vol. 186, N° 2, Juillet 1992, pp 977-983 ; (5) "Selective High Affinity RAR-α or RAR-β Retinoic Acid Receptor Ligands" *in* Mol. Pharmacol., Vol 40, pp 556-562.

Les antagonistes de l'acide rétinoïque sont notamment des composés décrits dans les demandes de brevet EP 661 259, EP 658 553, EP 568 898, WO 95/33745 et WO 94/14777, et dans plusieurs publications scientifiques, notamment EYROLLES & al (J. Med. Chem., 37, 1994, 1508-1517; Med. Chem. Res., 2, 1992, 361-367) et KANEKO & al (Med. Chem. Res., 1, 1991, 220-225) qui inhibent l'action de l'acide rétinoïque et/ou des retinoïdes.

Les antagonistes RARs utiles selon l'invention sont en particulier sélectionnés parmi les composés suivants:
- acide 4-[7-(1-adamantyl) -6-méthoxyéthoxyméthoxy -2-naphtyl] benzoïque,
- acide 4-[7-(1-adamantyl) -6-méthoxyéthoxyméthoxy -2-naphtyl] salicylique,
- acide 4-{[5,6-dihydro -5,5-diméthyl -8-(4-méthylphényl) -2-naphtalenyl] éthynyl} benzoïque,
- acide (E)-4-[2-(4,4-diméthyl -7-heptyloxy -1,1-dioxo -3,4-dihydro -2H-1-benzothiopyran -6-yl) propenyl] benzoïque,
- acide 4-[4,5,7,8,9,10-hexahydro -7,7,10,10-tétraméthyl -1-(3-pyridylméthyl) anthra[1,2-b]pyrrol -3-yl] benzoïque,
- acide 4-[4,5,7,8,9,10-hexahydro -7,7,10,10-tétraméthyl -1-(3-pyridylméthyl) thioanthra[1,2-b]pyrrol -3-yl] benzoïque,
- acide 4-[4,5,7,8,9,10-hexahydro -7,7,10,10-tétraméthyl -1-(3-pyridylméthyl} anthra{1,2-d]pyrazol -3-yl] benzoïque,
- acide 4-[3-(diamantyl) -4-méthoxybenzamido] benzoïque,
- acide 4-[3-(diamantyl) -4-méthoxybenzoyloxy] benzoïque,
- acide 4-(N-phényl -5,6,7,8-tétrahydro -5,5,8,8-tétraméthylnaphto[2,3-d]imidazol-2-yl)benzoïque,
- acide 4-(N-benzyl -5,6,7,8-tétrahydro -5,5,8,8-tétraméthylnaphto[2,3-d]imidazol-2-yl)benzoïque,
- acide 4-(5H-7,8,9,10-tétrahydro -5,7,7,10,10-pentaméthylbenzo[c] -naphto[2,3-b] [1,4]diazepin -3-yl)benzoïque,
- acide 4-[1-(1-méthoxy-2,2,2-trifluoroéthyl) -5,6,7,8-tétrahydro -5,5,8,8-tétraméthyl -3-anthracényl]benzoïque,
- acide 7-[3-(1-adamantyl)-4-méthoxyphenyl]-3,7-diméthyl-2,4,6-heptatrienoïque,
- acide 6-[7-(1-adamantyl)-6-méthoxyéthoxyméthoxy-2-naphtyl]nicotinique.
- acide 4-(5,5-diméthyl-8-phényl-5,6-dihydro-naphtalen-2-yléthynyl)benzoïque,
- acide 4-(5,5-diméthyl-8-p-tolyl-5,6-dihydro-naphtalen-2-yléthynyl)benzoïque.

De préférence, on utilise l'acide 4-[7-(1-adamantyl)-6-méthoxyéthoxyméthoxy-2-naphtyl]benzoïque, l'acide 4-[7-(1-adamantyl)-6-méthoxyéthoxyméthoxy-2-naphtyl]salicylique, l'acide 7-[3-(1-adamantyl)-4-méthoxyphenyl]-3,7-diméthyl-2,4,6-heptatrienoïque ou l'acide 6-[7-(1-adamantyl)-6-méthoxyéthoxyméthoxy-2-naphtyl]nicotinique.

La composition cosmétique ou pharmaceutique comprenant au moins un inhibiteur de l'activité de l'acide rétinoïque comprend un support cosmétiquement ou pharmaceutiquement acceptable et compatible avec le mode d'administration retenu.

La quantité d'inhibiteur de l'activité de l'acide rétinoïque est une quantité efficace dépendant bien entendu du traitement désiré et de la nature du composé choisi ; elle est donc déterminée par l'homme du métier.

L'administration des composés selon l'invention peut être effectuée par voie systémique (notamment entérale ou parentérale), topique ou oculaire.

Par voie entérale, la composition est plus particulièrement une composition pharmaceutique, pouvant se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, la composition, plus particulièrement la composition pharmaceutique, peut se présenter sous forme de solutions, d'émulsions ou de suspensions pour perfusion ou pour injection.

Les inhibiteurs de l'activité de l'acide rétinoïque, plus particulièrement les antagonistes de l'acide rétinoïque, sont généralement administrés par voie systémique à une dose journalière d'environ 0,01 mg/kg à 100 mg/kg en poids corporel, et ceci à raison de 1 à 3 prises.

Par voie topique, la composition est une composition cosmétique ou pharmaceutique, plus particulièrement destinée au traitement de la peau. Elle peut alors se présenter sous forme de solutions ou de suspensions, d'onguents, de pommades, de crèmes, de laits, de gels, de poudres, de tampons imbibés, de lotions, de sprays, ou de produits moussants ou nettoyants. Elle peut également se présenter sous forme de microsphères ou nanosphères ou vésicules lipidiques ou polymériques ou de patches polymériques, d'hydrogels ou de pansements permettant une libération contrôlée. Cette composition par voie topique peut par ailleurs se présenter soit sous forme anhydre, soit sous une forme aqueuse.

Elle peut être également utilisée sur les cheveux, afin de traiter le cuir chevelu, sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

L'inhibiteur de l'activité de l'acide rétinoïque peut être aussi incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Par voie oculaire, la composition peut alors se présenter sous forme d'onguents, de crèmes, de gels ou de collyres appropriés pour cette application spécifique.

Cette composition à usage topique ou oculaire contient au moins un antagoniste de l'acide rétinoïque à une concentration de préférence allant de 0,000001% à 5% en poids par rapport au poids total de la composition, plus particulièrement, allant de 0,0001% à 0,1% en poids.

La composition selon l'invention peut en outre contenir des additifs inertes ou même pharmacodynamiquement ou cosmétiquement actifs ou des combinaisons de ces additifs, et notamment : des agents mouillants ; des émollients; des agents hydratants comme le glycérol, le PEG 400, la thiamorpholinone, et ses dérivés ou bien encore l'urée ; des caroténoïdes et, notamment, le β-carotène.

La composition peut également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des agents photoprotecteurs, tels que des filtres UV-A et/ou UV-B, des antioxydants hydrophiles ou lipophiles, tels que l'α-tocophérol, le butylhydroxyanisole ou le butylhydroxytoluène, des chélatants comme l'EDTA et ses sels.

L'inhibiteur de l'activité de l'acide rétinoïque et les autres composés actifs peuvent être administrés ensemble dans une même composition cosmétique ou pharmaceutique, ou encore séparément dans des compositions séparées, de manière simultanée ou décalée dans le temps.

Les quantités des différents constituants des compositions selon l'invention sont celles classiquement utilisées dans les domaines considérés.

Bien entendu, la nature et la quantité de ces constituants sont choisies pour ne pas venir contrarier l'activité de l'inhibiteur de l'activité de l'acide rétinoïque pour traiter les peaux sensibles et/ou des dommages cutanés aigus induits par les rayonnements U.V..

On peut entre autres associer les inhibiteurs de l'activité de l'acide rétinoïque à des agents actifs destinés notamment à la prévention et/ou au traitement d'affections cutanées.

Parmi ces agents actifs, on peut citer à titre d'exemple :
- les agents modulant la différenciation et/ou la prolifération et/ou la pigmentation cutanée tels que la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme le peroxyde de benzoyle.

De façon avantageuse, les inhibiteurs de l'activité de l'acide rétinoïque sont associés à des actifs à effet secondaire irritant utilisés couramment dans le domaine cosmétique ou dermatologique. La présence d'un inhibiteur dans une composition cosmétique ou dermatologique contenant un actif ayant un effet irritant permet d'atténuer fortement, voire de supprimer cet effet.

Aussi, l'invention a encore pour objet une composition contenant un milieu cosmétiquement ou pharmaceutiquement acceptable et au moins un actif à effet secondaire irritant, à l'exception de l'acide rétinoïque et des rétinoïdes, caractérisée en ce qu'elle comprend au moins un inhibiteur de l'activité de l'acide rétinoïque.

En particulier, les actifs à effet secondaire irritant sont choisis parmi les α-hydroxy-acides, les β-hydroxy-acides, les α-céto-acides, les β-céto-acides, les anthralines, les anthranoïdes, les peroxydes, le minoxidil, les sels de lithium, les antimétabolites, la vitamine D et ses dérivés.

Du fait de son intérêt dans le domaine solaire, la présente invention a encore pour objet une composition contenant un milieu cosmétiquement ou pharmaceutiquement acceptable et au moins un agent photoprotecteur, caractérisée en ce qu'elle comprend au moins un inhibiteur de l'activité de l'acide rétinoïque.

A titre d'agent photoprotecteur, on peut utiliser les filtres organiques qui sont des filtres solaires classiques, actifs dans l'UV-A et/ou l'UV-B, hydrophiles ou lipophiles. A titre d'exemples, ces filtres peuvent être choisis, seuls ou en mélange, parmi l'acide 2-phényl benzimidazole 5-sulfonique et ses sels, les dérivés cinnamiques, les dérivés salicyliques, les dérivés du benzylidène camphre, les dérivés de triazine, les dérivés de la benzophénone, les dérivés du dibenzoylméthane, les dérivés de β,β-diphénylacrylate, les dérivés de l'acide p-aminobenzoïque, l'anthranilate de menthyle, les polymères filtres et silicones filtres décrits dans la demande WO-93-04665. D'autres exemples de filtres organiques sont donnés dans la demande de brevet EP-A 0 487 404.

Le ou les filtres organiques sont généralement présents dans les compositions selon l'invention à une teneur allant de 0,1 % à 30 %, de préférence de 0,5 à 15 %, en poids, par rapport au poids total de la composition.

Une deuxième catégorie d'agents photoprotecteurs convenant particulièrement bien aux compositions selon l'invention est celle des pigments. De préférence, on met en oeuvre des nanopigments (taille moyenne des particules primaires : généralement entre 5 nm et 100 nm, de préférence entre 10 et 50 nm) minéraux d'oxydes métalliques enrobés ou non comme par exemple des nanopigments d'oxyde de titane (amorphe ou cristallisé sous forme rutile et/ou anatase), de fer, de zinc, de zirconium ou de cerium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium ou encore les silicones. De tels nanopigments d'oxydes métalliques, enrobés ou non enrobés, sont en particulier décrits dans les demandes de brevets EP-A-0 518 772 et EP-A-0 518 773.

Le ou les (nano)pigments minéraux peuvent être présents dans les compositions selon l'invention à une teneur comprise entre 0,1 % et 30 %, de préférence de 0,5 % à 10 %, en poids, par rapport au poids total de la composition.

La présente invention a en outre pour objet un procédé de traitement cosmétique, caractérisé par le fait que l'on applique sur la peau et/ou sur les cheveux une composition telle que décrite ci-dessus contenant au moins un inhibiteur de l'activité de l'acide rétinoïque dans un milieu cosmétiquement acceptable.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings.

On va maitenant donner, à titre d'illustration, plusieurs exemples.

### EXEMPLE 1

Etude de l'activité des inhibiteurs de l'activité de l'acide rétinoïque pour protéger la peau contre les effets biologiques délétères aigus induits par les rayonnements U.V..

Cet exemple à pour but de mettre en évidence l'activité d'un antagoniste RAR dans la protection de la peau contre les effets biologiques cutanés aigus induits par l'irradiation UVB.

### Méthode

Pour ces études, un tube UVB présentant un pic à 315 nm est utilisé.
Une irradiation unique à la dose de 120 mJ/cm² est pratiquée sur l'oreille chez des souris Balb/c. L'irradiation est pratiquée sous anesthésie, les animaux, hors zone irradiée, étant protégés par un cache.

L'acide 4-[7-(1-adamantyl)-6-méthoxyéthoxyméthoxy-2-naphtyl]benzoïque (antagoniste RAR) en solution dans l'acétone aux concentrations de 0.01 %, 0.03 % et 0.1 %, ou le véhicule seul, est administré en application topique sur l'oreille irradiée, sous un volume de 20 microlitres.

Le traitement est administré 30 minutes, 24 heures, 48 heures et 72 heures après irradiation.

Chaque jour pendant 11 jours après l'irradiation (de J1 à J11), des observations cliniques sont effectuées et l'oedème est quantifié en mesurant l'épaisseur de l'oreille à l'aide d'un micromètre.

### Résultats

Chez les animaux irradiés et non traités, ou traités avec le véhicule seul, on observe un érythème et un oedème cliniquement visible 24 heures après irradiation et atteignant une valeur maximum 9 jours après irradiation. Une desquamation apparaît 4 jours après irradiation. Ce phénomène s'accentue durant la deuxième semaine après l'irradiation.

Chez les animaux traités avec l'acide 4-[7-(1-adamantyl)-6-méthoxyéthoxyméthoxy-2-naphtyl]benzoïque, on observe une diminution dose-dépendante de l'érythème, de l'oedème et de la desquamation. La valeur de l'aire sous la courbe de l'épaisseur de l'oreille déterminée par la méthode des trapèzes sur la période J1-J11 est diminuée de manière statistiquement significative par le traitement aux doses de 0.03 % et 0.1 %, respectivement de 26 % et de 55 %.

Ces résultats montrent clairement que les inhibiteurs de l'activité de l'acide rétinoïque ou de ses métabolites, et plus particulièrement les antagonistes RARs présentent une activité réparatrice des effets biologiques délétères aigus induits par l'irradiation UVB.

### EXEMPLE 2

Etude de l'évaluation des inhibiteurs de l'acide rétinoïque pour protéger la peau contre les effets délétères aigus induits par les rayonnements UV.

Cet exemple à pour but de mettre en évidence l'activité d'un antagoniste RAR dans la protection de la peau contre les effets biologiques cutanés aigus induits par l'irradiation UVB.

### Méthode

De la peau humaine provenant d'un donneur sain est transplantée chez la souris nude selon la méthode décrite par DEMARCHEZ & al (M.DEMARCHEZ & al, Develop. Biology, 113, 1986, 90-96; M.DEMARCHEZ & al, Develop. Biology, 121, 1987, 119 - 129).
Cinq mois après la transplantation, une irradiation unique à la dose de 300 mJ/cm² est pratiquée sur la moitié du greffon à l'aide d'une lampe UVB présentant un pic à 315 nanomètres. L'autre moitié du greffon protégée par un cache durant l'irradiation est utilisée comme contrôle intra-individuel.
L'acide 4 - [7 - (1 adamantyl) - 6 - méthoxyéthoxyméthoxy - 2 naphtyl] benzoïque (antagoniste RAR) en solution dans l'acétone à la concentration de 0.1 %, ou le véhicule seul, est administré en application topique sur le greffon le jour de l'irradiation puis une fois par jour les quatre jours suivant l'irradiation.
24 heures après le dernier traitement les souris sont euthanasiées et la peau des greffons est prélevée et fixée par congélation pour étude immunohistochimique. Différents anticorps sont utilisés pour étudier l'effet des UVB et des traitements sur la morphologie cutanée: anti - involucrine humaine (étude de la différenciation épidermique), AE1 (anticorps monoclonal anti - kératines), anti - HLADr (cellules de Langerhans).

### Résultats

L'irradiation UVB induit dans l'épiderme des greffons traités avec le véhicule seul une légère hyperplasie, une hypergranulose, une parakératose, une perturbation de la différenciation épidermique et des « sun burn cells ».

Le traitement avec l'acide 4 - [7 - (1 adamantyl) - 6 - méthoxyéthoxyméthoxy - 2 naphtyl] benzoïque limite l'hypergranulose et l'hyperkératose, et diminue le nombre de « sun burn cells ».

Ces résultats montrent clairement que les inhibiteurs de l'activité de l'acide rétinoïque ou de ses métabolites, et plus particulièrement les antagonistes RAR présentent une activité réparatrice des effets biologiques délétères aigus induits par l'irradiation UVB.

### EXEMPLE 3

Etude de l'évaluation des inhibiteurs de l'acide rétinoïque comme traitement des peaux sensibles.

Cet exemple à pour but de mettre en évidence l'activité d'un antagoniste RAR comme régulateur de l'homéostasie cutanée dans les zones de bordure de greffe.

### Méthode

De la peau humaine provenant d'un donneur sain est transplantée chez la souris nude selon la méthode décrite par DEMARCHEZ & al (M.DEMARCHEZ & al, Develop. Biology, 113, 1986, 90-96; M.DEMARCHEZ & al, Develop. Biology, 121, 1987, 119 - 129).
Il est bien établi que dans ce modèle de peau humaine transplantée chez la souris nude la périphérie des greffons est une zone particulièrement sensible aux agents chimiques comme les produits irritants ou aux agents physiques comme les UV. Ainsi, cette zone périphérique peut être considérée comme modèle de la peau sensible.

Trois mois après transplantation, l'acide 4 - [7 - (1 adamantyl) - 6 - méthoxyéthoxyméthoxy - 2 naphtyl] benzoïque (antagoniste RAR), en solution dans l'acétone à la concentration de 0.1 %, ou le véhicule seul, est administré en application topique sur le greffon une fois par jour pendant six semaines.
24 heures après le dernier traitement les souris sont euthanasiées et la peau des greffons est prélevée et fixée par congélation pour étude immunohistochimique. Différents anticorps sont utilisés pour étudier l'effet des UVB et des traitements sur la morphologie cutanée: anti - involucrine humaine (étude de la différenciation épidermique), AE1 (anticorps monoclonal anti - kératines).

### Résultats

Les greffons traités avec le véhicule seul présentent dans la zone de bordure de greffe une perturbation de la différenciation épidermique.

L'acide 4 - [7 - (1 adamantyl) - 6 - méthoxyéthoxyméthoxy - 2 naphtyl] benzoïque ne modifie pas l'aspect histologique des greffons excepté au niveau des bordures de greffes où il régule la différenciation épidermique.

Ces résultats montrent clairement que les inhibiteurs de l'activité de l'acide rétinoïque ou de ses métabolites, et plus particulièrement les antagonistes RAR, présentent une activité modulatrice de l'homéostasie cutanée dans les peaux sensibles.

### EXEMPLE 4

Etude de l'évaluation des inhibiteurs de l'acide rétinoïque comme traitement des peaux sensibles.

Cet exemple à pour but de mettre en évidence l'activité d'un antagoniste RAR comme régulateur de l'homéostasie cutanée dans les zones de bordure de greffe.

### Méthode

De la peau humaine provenant d'un donneur sain est transplantée chez la souris nude selon la méthode décrite par DEMARCHEZ & al (M.DEMARCHEZ & al, Develop. Biology, 113, 1986, 90-96; M.DEMARCHEZ & al, Develop. Biology, 121, 1987, 119 - 129)
Il est bien établi que dans ce modèle de peau humaine transplantée chez la souris nude la périphérie des greffons est une zone particulièrement sensible aux agents chimiques comme les produits irritants ou aux agents physiques comme les UV. Ainsi cette zone périphérique peut être considérée comme modèle de la peau sensible.

Cinq mois après transplantation, l'acide 4 - [7 - (1 adamantyl) - 6 - méthoxyéthoxyméthoxy - 2 naphtyl] benzoïque (antagoniste RAR), ou l'acide 4 - [7 - (1 adamantyl) - 6 - méthoxyéthoxyméthoxy - 2 naphtyl] salicylique en suspension dans le crémophore EL 25 %, ou le véhicule seul, est administré oralement une fois par jour pendant 7 jours.
24 heures après le dernier traitement les souris sont euthanasiées et la peau des greffons est prélevée et fixée par congélation pour étude immunohistochimique. Différents anticorps sont utilisés pour étudier l'effet des traitements sur la morphologie cutanée: anti - involucrine humaine (étude de la différenciation épidermique), AE1 (anticorps monoclonal anti - kératines).

### Résultats

Les greffons traités avec le véhicule seul présentent dans la zone de bordure de greffe une perturbation de la différenciation épidermique.

L'acide 4 - [7 - (1 adamantyl) - 6 - méthoxyéthoxyméthoxy - 2 naphtyl] benzoïque et l'acide 4 - [7 - (1 adamantyl) - 6 - méthoxyéthoxyméthoxy - 2 naphtyl] salicylique ne modifient pas l'aspect histologique des greffons excepté au niveau des bordures de greffes où il régule la différenciation épidermique.

Ces résultats montrent clairement que les inhibiteurs de l'activité de l'acide rétinoïque ou de ses métabolites, et plus particulièrement les antagonistes RAR, présentent une activité modulatrice de l'homéostasie cutanée dans les peaux sensibles.

### EXEMPLE 5

Les compositions ci-après sont des exemples de compositions susceptibles d'être employées pour appliquer ou administrer des antagonistes de l'acide rétinoïque dans le but de traiter les peaux sensibles et/ou les dommages aigus dus aux rayonnements U.V..

### A- VOIE ORALE

(a) Comprimé 0,2 g à libération immédiate (granulation)
   - Antagoniste de l'acide rétinoïque 0,0010 g
   - Lactose codex 0,1204 g
   - Cellulose microcristalline (Avicel PH 101) 0,0640 g
   - Polyvinyl-pyrrolidone (Kollidon K30) 0,0060 g
   - Silice colloïdale (Aerosil 200) 0,0006 g
   - Stéarate de magnésium 0,0020 g
   - Glycolate d'amidon sodique (Explotab) 0,0060 g
   - Eau purifiée qsp granulation
(b) Comprimé 0,8 g à libération immédiate (compression directe)
   - Antagoniste de l'acide rétinoïque 0,0800 g
   - Amidon modifié (Starch 1500) 0,2984 g
   - Cellulose microcristalline (Avicel PH 102) 0,4000 g
   - Silice colloïdale (Aerosil 200) 0,0016 g
   - Sréarate de magnésium 0,0040 g
   - Croscarmellose sodique (Ac-Di-Sol) 0,0160 g
(c) Gélule 0,2 g (capsule molle ou gélule remplie de liquide)
   - Antagoniste de l'acide rétinoïque 0,0050 g
   - Glycérides polyglycosylés insaturés ou huile végétale (soja, maïs, ...) 0,1845 g
   - Cire blanche d'abeille ou huile de ricin hydrogénée 0,0100 g
   - BHT 0,0001 g
   - dl-α-tocophérol 0,0004 g
(d) Solution buvable en ampoules de 5 ml
   - Antagoniste de l'acide rétinoïque 0,0001 g
   - Glycérol 0,7500 g
   - Sorbitol 1,0000 g
   - Propylène glycol 1,0000 g
   - Polyvinyl pyrrolidone (Kollidon K25) 0,5000 g
   - Cyclamate de sodium 0,0050 g
   - Parahydroxybenzoate de sodium 0,0040 g
   - Arome qs
   - Eau purifiée qsp 5 ml

### B- VOIE TOPIQUE

(a) Onguent
   - Antagoniste de l'acide rétinoïque 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Antagoniste de l'acide rétinoïque 0,300 g
   - Vaseline blanche codex qsp 100 g
(c) Crème Eau-dans-Huile non ionique
   - Antagoniste de l'acide rétinoïque 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Antagoniste de l'acide rétinoïque 0,100 g
   - Polyéthylène glycol (PEG 400) 69,900 g
   - Ethanol à 95% 30,000 g
(e) Crème Huile-dans-Eau non ionique
   - Antagoniste de l'acide rétinoïque 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Utilisation d'au moins un inhibiteur de l'activité de l'acide rétinoïque dans une composition cosmétique ou pour la préparation d'une composition pharmaceutique, l'inhibiteur de l'activité de l'acide rétinoïque ou la composition pharmaceutique étant destiné à traiter les peaux sensibles et/ou les dommages cutanés aigus induits par les rayonnements U.V..

2. Utilisation d'au moins un inhibiteur de l'activité de l'acide rétinoïque dans une composition cosmétique ou pour la préparation d'une composition pharmaceutique, l'inhibiteur de l'activité de l'acide rétinoïque ou la composition pharmaceutique étant destiné à traiter les irritations cutanées et/ou les érythèmes et/ou les rougeurs si/ou les sensations dysesthésiques et/ou les sensations d'échauffement et/ou les prurits et/ou les picotements et/ou les fourmillements et/ou les démangeaisons et/ou les tiraillements de la peau.

3. Utilisation selon la revendication précédente, **caractérisée en ce que** l'inhibiteur de l'activité de l'acide rétinoïque ou la composition pharmaceutique est destiné à traiter les érythèmes et/ou les irritations et/ou les rougeurs et/ou les gonflements et/ou les oedèmes et/ou la desquamation dus aux rayonnements U.V..

4. Utilisation selon l'une des revendications 1 à 3, **caractérisée en ce que** la composition pharmaceutique est une composition dermatologique.

5. Utilisation selon l'une quelconque des revendications précédentes, **caractérisée en ce que** l'inhibiteur de l'activité de l'acide rétinoïque ou la composition pharmaceutique est destiné à traiter de manière curative.

6. Utilisation selon l'une quelconque des revendications précédentes, **caractérisée en ce que** l'inhibiteur de l'activité de l'acide rétinoïque est un antagoniste RARs.

7. Utilisation selon la revendication précédente, **caractérisée en ce que** les antagonistes RARs sont choisis parmi les composés suivants:
- acide 4-[7-(1-adamantyl) -6-méthoxyéthoxyméthoxy -2-naphtyl] benzoïque,
- acide 4-[7-(1-adamantyl) -6-méthoxyéthoxyméthoxy -2-naphtyl]salicylique,
- acide 4-{[5,6-dihydro -5,5-diméthyl -8-(4-méthylphényl) -2-naphtalenyl] éthynyl} benzoïque,
- acide (E)-4-[2-(4,4-diméthyl -7-heptyloxy -1,1-dioxo -3,4-dihydro -2H-1-benzothiopyran -6-yl) propenyl] benzoïque,
- acide 4-[4,5,7,8,9,10-hexahydro -7,7,10,10-tétraméthyl -1 -(3-pyridylméthyl) anthra[1,2-b]pyrrol -3-yl] benzoïque,
- acide 4-[4,5,7,8,9,10-hexahydro -7,7,10,10-tétraméthyl -1-(3-pyridylméthyl) thioanthra[1,2-b]pyrrol -3-yl] benzoïque,
- acide 4-[4,5,7,8,9,10-hexahydro -7,7,10,10-tétraméthyl -1-(3-pyridylméthyl) anthra[1,2-d]pyrazol -3-yl] benzoïque,
- acide 4-[3-(diamantyl) -4-méthoxybenzamido] benzoïque,
- acide 4-[3-(diamantyl) -4-méthoxybenzoyloxy] benzoïque,
- acide 4-(N-phényl -5,6,7,8-tétrahydro -5,5,8,8-tétraméthylnaphto[2,3-d]imidazol-2-yl)benzoïque,
- acide 4-(N-benzyl -5,6,7,8-tétrahydro -5,5,8,8-tétraméthylnaphto[2,3-d]imidazol-2-yl)benzoïque,
- acide 4-(5H-7,8,9,10-tétrahydro -5,7,7,10,10-pentaméthylbenzo[c] -naphto[2,3-b] [1,4]diazepin -3-yl)benzoïque,
- acide 4-[1-(1-méthoxy-2,2,2-trifluoroéthyl) -5,6,7,8-tétrahydro -5,5,8,8-tétraméthyl -3-anthracényl]benzoïque,
- acide 7-[3-(1-adamantyl)-4-méthoxyphenyl]-3,7-diméthyl-2,4,6-heptatrienoïque,
- acide 6-[7-(1-adamantyl)-6-méthoxyéthoxyméthoxy-2-naphtyl]nicotinique,
- acide 4-(5,5-diméthyl-8-phényl-5,6-dihydro-naphtalen-2-yléthynyl)benzoïque,
- acide 4-(5,5-diméthyl-8-p-tolyl-5,6-dihydro-naphtalen-2-yléthynyl)benzoïque.

8. Utilisation selon la revendication précédente, **caractérisée en ce que** les antagonistes RARs sont choisis parmi :
- acide 4-[7-(1-adamantyl)-6-méthoxyéthoxyméthoxy-2-naphtyl]benzoïque,
- acide 4-[7-(1-adamantyl)-6-méthoxyéthoxyméthoxy-2-naphtyl]salicylique,
- acide 7-[3-(1-adamantyl)-4-méthoxyphenyl)-3,7-diméthyl-2,4,6-heptatrienoïque,
- acide 6-[7-(1-adamantyl)-6-méthoxyéthoxyméthoxy-2-naphtyl]nicotinique.

## Claims

1. Use of at least one inhibitor of the activity of retinoic acid in a cosmetic composition or for the preparation of a pharmaceutical composition, the inhibitor of the activity of retinoic acid or the pharmaceutical composition being intended for treating sensitive skins and/or the acute cutaneous damage induced by UV radiation.

2. Use of at least one inhibitor of the activity of retinoic acid in a cosmetic composition or for the preparation of a pharmaceutical composition, the inhibitor of the activity of retinoic acid or the pharmaceutical composition being intended for treating cutaneous irritation and/or erythema and/or reddening and/or dysaesthetic sensations and/or sensations of inflammation and/or pruritus and/or pricking and/or formication and/or itching and/or twitching of the skin.

3. Use according to the preceding claim, **characterized in that** the inhibitor of the activity of retinoic acid or the pharmaceutical composition is intended for treating erythema and/or irritation and/or reddening and/or swelling and/or oedema and/or desquamation due to UV radiation.

4. Use according to one of Claims 1 to 3, **characterized in that** the pharmaceutical composition is a dermatological composition.

5. Use according to any one of the preceding claims, **characterized in that** the inhibitor of the activity of retinoic acid or the pharmaceutical composition is intended to treat in a curative manner.

6. Use according to any one of the preceding claims, **characterized in that** the inhibitor of the activity of retinoic acid is an RAR antagonist.

7. Use according to the preceding claim, **characterized in that** the RAR antagonists are chosen from the following compounds:
- 4-[7-(1-adamantyl)-6-methoxyethoxymethoxy-2-naphthyl]benzoic acid,
- 4-[7-(1-adamantyl)-6-methoxyethoxymethoxy-2-naphthyl]salicylic acid,
- 4-{[5,6-dihydro-5,5-dimethyl-8-(4-methylphenyl)-2-naphthalenyl]ethynyl}benzoic acid,
- (E)-4-[2-(4,4-dimethyl-7-heptyloxy-1,1-dioxo-3,4-dihydro-2H-1-benzothiopyran-6-yl)propenyl]benzoic acid,
- 4-[4,5,7,8,9,10-hexahydro-7,7,10,10-tetramethyl-1-(3-pyridylmethyl)anthra[1,2-b]pyrrol-3-yl]benzoic acid,
- 4-[4,5,7,8,9,10-hexahydro-7,7,10,10-tetramethyl-1-(3-pyridylmethyl)thioanthra[1,2-b]pyrrol-3-yl]benzoic acid,
- 4-[4,5,7,8,9,10-hexahydro-7,7,10,10-tetramethyl-1-(3-pyridylmethyl)anthra[1,2-d]pyrazol-3-yl]benzoic acid,
- 4-[3-(diamantyl)-4-methoxybenzamido]benzoic acid,
- 4-[3-(diamantyl)-4-methoxybenzoyloxy]benzoic acid,
- 4-(N-phenyl-5,6,7,8-tetrahydro-5,5,8,8-tetramethylnaphtho[2,3-d]imidazol-2-yl)benzoic acid,
- 4-(N-benzyl-5,6,7,8-tetrahydro-5,5,8,8-tetramethylnaphtho[2,3-d]imidazol-2-yl)benzoic acid,
- 4-(5H-7,8,9,10-tetrahydro-5,7,7,10,10-pentamethylbenzo[c]-naphtho[2,3-b][1,4]diazepin-3-yl)benzoic acid,
- 4-[1-(1-methoxy-2,2,2-trifluoroethyl)-5,6,7,8-tetrahydro-5,5,8,8-tetramethyl-3-anthracenyl]benzoic acid,
- 7-[3-(1-adamantyl)-4-methoxyphenyl]-3,7-dimethyl-2,4,6-heptatrienoic acid,
- 6-[7-(1-adamantyl)-6-methoxyethoxymethoxy-2-naphthyl]nicotinic acid,
- 4-(5,5-dimethyl-8-phenyl-5,6-dihydronaphthalen-2-ylethynyl)benzoic acid,
- 4-(5,5-dimethyl-8-p-tolyl-5,6-dihydronaphthalen-2-ylethynyl)benzoic acid.

8. Use according to the preceding claim, **characterized in that** the RAR antagonists are chosen from:
- 4-[7-adamantyl)-6-methoxyethoxymethoxy-2-naphthyl]benzoic acid,
- 4-[7-(1-adamantyl)-6-methoxyethoxymethoxy-2-naphthyl]salicylic acid,
- 7-[3-(1-adamantyl)-4-methoxyphenyl]-3,7-dimethyl-2,4,6-heptatrienoic acid,
- 6-[7-(1-adamantyl)-6-methoxyethoxymethoxy-2-naphthyl]nicotinic acid.

## Patentansprüche

1. Verwendung mindestens eines Inhibitors der Aktivität der Retinsäure in einer kosmetischen Zusammensetzung oder zur Herstellung einer pharmazeutischen Zusammensetzung, wobei der Inhibitor der Aktivität der Retinsäure oder die pharmazeutische Zusammensetzung zur Behandlung von empfindlicher Haut und/oder von durch UV-Strahlung hervorgerufenen akuten Hautschäden bestimmt ist.

2. Verwendung mindestens eines Inhibitors der Aktivität der Retinsäure in einer kosmetischen Zusammensetzung oder zur Herstellung einer pharmazeutischen Zusammensetzung, wobei der Inhibitor der Aktivität der Retinsäure oder die pharmazeutische Zusammensetzung zur Behandlung von Hautreizungen und/oder Erythemen und/oder Rötungen und/oder Dysästhesien und/oder Hitzeempfindungen der Haut und/oder Pruriti und/oder Prickeln und/oder Kribbeln und/oder Jucken und/oder Ziehen der Haut bestimmt ist.

3. Verwendung nach dem vorhergehenden Anspruch, **dadurch gekennzeichnet, daß** der Inhibitor der Aktivität der Retinsäure oder die pharmazeutische Zusammensetzung zur Behandlung von Erythemen und/oder Reizungen und/oder Rötungen und/oder Schwellungen und/oder Ödemen und/oder von durch UV-Strahlung bedingten Abschuppungen bestimmt ist.

4. Verwendung nach einem der Ansprüche 1 bis 3, **dadurch gekennzeichnet, daß** die pharmazeutische Zusammensetzung eine dermatologische Zusammensetzung ist.

5. Verwendung nach einem der vorhergehenden Ansprüche, **dadurch gekennzeichnet, daß** der Inhibitor der Aktivität der Retinsäure oder die pharmazeutische Zusammensetzung zur kurativen Behandlung bestimmt ist.

6. Verwendung nach einem der vorhergehenden Ansprüche, **dadurch gekennzeichnet, daß** der Inhibitor der Aktivität der Retinsäure ein RAR-Antagonist ist.

7. Verwendung nach dem vorhergehenden Anspruch, **dadurch gekennzeichnet, daß** die RAR-Antagonisten unter folgenden Verbindungen ausgewählt sind:
• 4-[7-(1-Adamantyl)-6-methoxyethoxymethoxy-2-naphthyl]-benzoesäure,
• 4-[7-(1-Adamantyl)-6-methoxyethoxymethoxy-2-naphthyl]-salicylsäure,
• 4-{[5,6-Dihydro-5,5-dimethyl-8-(4-methylphenyl)-2-naphthylenyl]-ethinyl}-benzoesäure,
• (E)-4-[2-(4,4-Dimethyl-7-heptyloxy-1,1-dioxo-3,4-dihydro-2H-1-benzothiopyran-6-yl)-propenyl]-benzoesäure,
• 4-[4,5,7,8,9,10-Hexahydro-7,7,10,10-tetramethyl-1-(3-pyridylmethyl)-anthra-[1,2-b]-pyrrol-3-yl]-benzoesäure,
• 4-[4,5,7,8,9,10-Hexahydro-7,7,10,10-tetramethyl-1-(3-pyridylmethyl)-thioanthra-[1,2-b]-pyrrol-3-yl]-benzoesäure,
• 4-[4,5,7,8,9,10-Hexahydro-7,7,10,10-tetramethyl-1-(3-pyridylmethyl)-anthra-[1,2-d]-pyrazol-3-yl]-benzoesäure,
• 4-[3-(Diamantyl)-4-methoxybenzamido]-benzoesäure,
• 4-[3-(Diamantyl)-4-methoxybenzoyloxy]-benzoesäure,
• 4-(N-Phenyl-5,6,7,8-tetrahydro-5,5,8,8-tetramethylnaphtho-[2,3-d]-imidazol-2-yl)-benzoesäure,
• 4-(N-Benzyl-5,6,7,8-tetrahydro-5,5,8,8-tetramethylnaphtho-[2,3-d]-imidazol-2-yl)-benzoesäure,
• 4-(5H-7,8,9,10-Tetrahydro-5,7,7,10,10-pentamethylbenzo-[c]-naphtho-[2,3-b]-[1,4]-diazepin-3-yl)-benzoesäure,
• 4-[1-(1-Methoxy-2,2,2-trifluorethyl)-5,6,7,8-tetrahydro-5,5,8,8-tetramethyl-3-anthracenyl]-benzoesäure,
• 7-[3-(1-Adamantyl)-4-methoxyphenyl]-3,7-dimethyl-2,4,6-heptatriensäure,
• 6[7-(1-Adamantyl)-6-methoxyethoxymethoxy-2-naphthyl]-nicotinsäure,
• 4-(5,5-Dimethyl-8-phenyl-5,6-dihydronaphthalin-2-yl-ethinyl)-benzoesäure,
• 4-(5,5-Dimethyl-8-p-tolyl-5,6-dihydronaphthalin-2-yl-ethinyl)-benzoesäure.

8. Verwendung nach dem vorhergehenden Anspruch, **dadurch gekennzeichnet, daß** die RAR-Antagonisten ausgewählt sind unter:
• 4-[7-(1-Adamantyl)-6-methoxyethoxymethoxy-2-naphthyl]-benzoesäure,
• 4-[7-(1-Adamantyl)-6-methoxyethoxymethoxy-2-naphthyl]-salicylsäure,
• 7-[3(1-Adamantyl)-4-methoxyphenyl]-3,7-dimethyl-2,4,6-heptatriensäure,
• 6-[7-(1-Adamantyl)-6-methoxyethoxymethoxy-2-naphthyl]-nicotinsäure.
